# EUROPÄISCHE PATENTSCHRIFT

(11) **EP 3 165 598 B1**
(45) Veröffentlichungstag und Bekanntmachung des Hinweises auf die Patenterteilung: **29.05.2024**
(21) Anmeldenummer: 16197234.4
(22) Anmeldetag: 04.11.2016
(51) Int. Cl.: C12M 1/107, C12M 1/00, C12M 1/26

(54) **VERFAHREN ZUM BETREIBEN EINER BIOGASANLAGE**
METHOD FOR OPERATING A BIOGAS ASSEMBLY
PROCÉDÉ DE FONCTIONNEMENT D'UNE INSTALLATION DE PRODUCTION DE BIOGAZ

(30) Priorität: 05.11.2015 DE 102015118988
(43) Veröffentlichungstag der Anmeldung: 10.05.2017
(73) Patentinhaber: DIGITANALOG Hard- und Software GmbH, 63829 Krombach (DE)
(72) Erfinder: ALBERT, Alfred, 63829 Kromberg (DE)
(74) Vertreter: Stoffregen, Hans-Herbert

(56) Entgegenhaltungen:
- EP-A1- 0 189 155
- EP-A2- 0 038 489
- EP-A2- 0 158 714
- EP-B1- 0 625 961
- DE-A1- 2 446 289
- DE-A1-102005 040 849
- DE-A1-102011 016 604

## Beschreibung

Die Erfindung bezieht sich auf ein Verfahren zum Betreiben einer Biogasanlage umfassend zumindest einen Fermenter, in dem Biogas durch Vergärung von dem Fermenter zugeführtem Substrat erzeugt wird, einen dem Fermenter nachgeordneten Gärrest zuzuführenden Separator zum Separieren von insbesondere unvergärtem Feststoff und Gärresten in wässriger Fraktion, wobei die wässrige Fraktion des Gärrestes in einem Belebungsbecken nachbehandelt wird, und in dem Belebungsbecken anfallende Suspension dem nachgeschalteten Absetzbecken zugeführt wird.

Biogasanlagen dienen zur Erzeugung von Biogas durch Vergärung von Rohstoffen, Pflanzen und landwirtschaftlichen Nebenprodukten oder Bioabfällen. Dabei sind Rohstoffe, die Zellulose und Lignozellulose enthalten, allerdings unter den in dem Fermenter herrschenden anaeroben Bedingungen nur schwer abbaubar.

Bei gut abbaubaren Substraten wird ein großer Teil in Biogas umgesetzt. Ein wässriges Gemisch aus schwer abbaubarem organischem Material sowie aus anorganischen Stoffen, dem sogenannten Gärrest bleibt, zurück. Mittels eines Separators können die nicht abbaubaren Bestandteile aus dem Gärrest abgesondert werden. Die verbleibende wässrige Fraktion kann in ihrer Masse ggfs. durch eine aerobe Nachbehandlung verringert werden.

Durch verschärfte gesetzgeberische Maßnahmen ist eine Düngung mit den wässrigen Gärresten, die auch als Biogasgülle bezeichnet werden können, nur noch in einigen Monaten im Jahr möglich, so dass erhöhte Lagerkapazitäten für eine Biogasanlage erforderlich sind. Auch führt die Emission von Ammoniak und eventuell Lachgas zu dem Zwang, Gegenmaßnahmen zu ergreifen.

Um die anfallende Gärrestmenge zu verringern, erfolgen vorzugsweise maschinelle Eingriffe, um die anfallende Biogasgülle zu reduzieren. Mittels Separatoren wie Zentrifugen, Schneckenpressen oder entsprechender Geräte kann der Trockenmassegehalt TS von 4 bis 6 % auf bis zu 30 % erhöht werden. In der Festphase verbleibt ein stark verringerter Anteil an Ammonium (NH₄⁺) zurück. Mehr als die Hälfte des Ammoniums befindet sich jedoch in dem abgetrennten Prozesswasser, also Filtrat. Durch die Auftrennung in zwei Fraktionen ist zwar ein Teil der Probleme gelöst, d. h. die Lagerkapazität und der Ammoniumgehalt werden verringert. Allerdings ist das Problem auf die wässrige Phase verschoben.

Belebtschlammverfahren, bei denen einem Belebungsbecken von einem Absetzbecken stammender Schlamm zugeführt wird, sind der EP 0 189 155 A1 und EP 0 625 961 B1 zu entnehmen.

Aus der EP 0 158 714 A2 ist ein Behälter zum Klären von mit Feststoffen beladenen Flüssigkeiten unter Verwendung von Flockungsmitteln bekannt.

Der vorliegenden Erfindung liegt die Aufgabe zu Grunde, ein Verfahren der eingangs genannten Art so weiterzubilden, dass die Biogasanlage energetisch günstig betrieben werden kann, wobei gleichzeitig erforderliche Lagerkapazitäten verringert werden können. Auch soll der Anteil an Ammonium (NH₄⁺) reduziert werden.

Zur Lösung der Aufgabe sieht die Erfindung verfahrensmäßig im Wesentlichen vor, dass in dem Absetzbecken vorhandenes überstehendes Wasser vollständig oder teilweise dem Belebungsbecken zugeführt wird.

Dabei zeichnet sich die Erfindung insbesondere auch dadurch aus, dass bei der biologischen Nachbehandlung entstandener oder aus der biologischen Nachbehandlung durch Filtration gewonnener Schlamm dem Fermenter zugeführt wird. Erfindungsgemäß werden aufbereitete Gärreste dem Fermenter zugeführt, quasi als Co-Substrat, so dass die Menge des dem Fermenter zugeführten Substrats an sich verringert werden kann. Ungeachtet dessen wird die Menge des erzeugten Biogases nicht verringert.

Wird demgegenüber dem Fermenter im Vergleich zum Stand der Technik die gleiche Menge an Substrat zugeführt, so erhöht sich der Ertrag des Biogases.

Erfindungsgemäß wird dem dem Belebungsbecken nachgeschalteten Absetzbecken überstehendes Wasser entnommen, das vollständig oder teilweise dem Belebungsbecken wieder zugeführt wird. Hierdurch ergibt sich der Vorteil, dass die in der wässrigen Fraktion im Belebungsbecken vorhandenen nicht gelösten Stoffe nur wenig oxidiert werden mit der Folge, dass der dem Fermenter wieder zuzuführende Schlamm weniger oxidiert ist als derjenige, bei dem nach dem klassischen Belebtschlammverfahren dem Belebungsbecken Rückführschlamm zugeführt wird. Oxidierter Schlamm bedeutet jedoch, dass der Methananteil im Biogas reduziert wird.

Durch das erfindungsgemäße Verfahren werden weniger Gärreste ausgebracht, so dass die Stickstoffbelastung reduziert wird und sich somit eine bessere Humusbilanz ergibt. Ferner ist der Vorteil gegeben, dass weniger Platz für die Gärrestelagerung erforderlich ist. Auch erfolgt eine Verringerung der Geruchsbelästigung durch Gärreste.

Durch die Filtration der biologisch behandelten Gärreste, also des Schlamms, ergibt sich des Weiteren der Vorteil, dass das Filtrat quasi als einleitfähiges Wasser zu bezeichnen ist. Das Filtrat kann zum Versickern im Untergrund, zum Verregnen, zum Einleiten in eine Kläranlage oder in ein Oberflächengewässer verwendet werden.

Ergänzend oder alternativ ist vorgesehen, dass bei der Sedimentation im Absetzbecken entstehendes überstehendes Wasser zum Versickern im Untergrund, zum Verregnen, zum Einleiten in eine Kläranlage oder in ein Gewässer verwendet wird.

Insbesondere ist vorgesehen, dass dem Fermenter filtrierter Schlamm mit einem TS-Gehalt zwischen 8 % und 25 %, insbesondere zwischen 10 % und 20 %, zugeführt wird.

Die Erfindung zeichnet sich auch dadurch aus, dass dem Fermenter im Absetzbecken angefallenes Sediment mit einem Trockengehalt zwischen 3 % ≤ TS ≤ 10 %, insbesondere 4 % ≤ TS ≤ 8 %, zugeführt wird.

Durch das erfindungsgemäße Verfahren wird in einer biologischen Stufe der NH₄⁺-Gehalt durch speziell konditionierte Mikroorganismen in mehreren Schritten zu elementarem Stickstoff umgewandelt, so dass eine umweltgerechte Lösung der Stickstoffentfernung sichergestellt ist. Zudem stellen die Mikroorganismen durch ihren Stickstoffumsatz sicher, dass CSB, TOC und andere Parameter so stark reduziert werden, dass das gereinigte Wasser optisch klar anfällt.

Messungen haben ergeben, dass die Reduzierung des TOC (total organischer Kohlenstoff)-Gehalts auf 100 bis 200 mg/l reduziert wird. Vor der biologischen Behandlung nach Verlassen des Fermenters beträgt der Anteil in etwa 3000 mg/l. Bezüglich des CSB-Gehalts konnte eine Reduzierung von 10.000 mg/l auf 250 bis 500 mg/l erreicht werden. Hinsichtlich des NH₄⁺-Gehaltes konnte eine Reduzierung von 4500 mg/l auf weniger als 50 mg/l festgestellt werden. Hinsichtlich der Phosphate erfolgte eine Reduzierung von 80 mg/l auf weniger als 10 mg/l.

Das Verfahren zeichnet sich auch zur Feststoffsedimentation feinster Partikel, vorzugsweise Partikel einer Größe zwischen 1 µm und 50 µm, aus, wobei in dem Absetzbecken mit in diesem vorhandenen Sediment und über diesem überstehendem Fluid in das überstehende Fluid zumindest ein die Sedimentation beeinflussendes Hilfsmittel zugegeben wird.

Bevorzugterweise sieht die Erfindung vor, dass als Hilfsmittel zumindest eine Substanz aus der Gruppe Metallsalz, insbesondere Aluminium- und/oder Eisensalz wie Chloride oder Sulfate, und/oder Polymere wie kationische und/oder anionische Polyacrylamidderivate verwendet wird.

Hervorzuheben ist des Weiteren, dass als Hilfsmittel eine Mischung bestehend aus oder enthaltend Aluminiumhydroxidchlorid und/oder kationische und/oder anionische Stärkederivate verwendet wird.

Insbesondere ist vorgesehen, dass als Hilfsmittel eine Mischung verwendet wird, die Aluminiumhydroxidchlorid enthält. Ergänzend können anionische und/oder kationische, gegebenenfalls anionische und kationische Stärkederivate verwendet werden. Bezüglich des Aluminiumhydroxidchlorids ist anzumerken, dass dieses eine Basizität zwischen 25 % und 95 % aufweisen sollte. Der Gewichtsanteil in der Mischung sollte mehr als 40 Gew.-% betragen, wobei Werte zwischen 80 Gew.-% und 97 Gew.-% zu bevorzugen sind. Sofern kationische und/oder anionische Stärkederivate in der Mischung enthalten sind, sollte der Gewichtsanteil zwischen 0 Gew.-% und 30 Gew.-%, bevorzugterweise zwischen 5 Gew.-% und 10 Gew.-%, liegen.

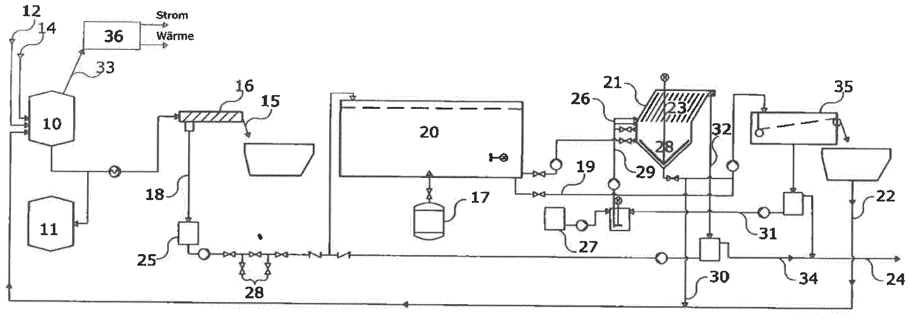

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung eines der Zeichnung zu entnehmenden bevorzugten Ausführungsbeispiels.

Der einzigen Fig. ist eine Prinzipdarstellung einer Biogasanlage zu entnehmen, bei der eine erfindungsgemäße Gärreste-Aufbereitung erfolgt. Die Anlage umfasst einen Fermenter 10, dem zur Biogaserzeugung über eine Leitung 12 Rohstoffe (Input-Material) zugeführt werden, die auch als Substrate oder Einsatzstoffe zu bezeichnen sind. Ferner können über eine Leitung 14 Additive beigegeben werden, um den mikrobiellen Abbau (Vergärung) des eingesetzten Substrats zu beeinflussen. Dem Fermenter 10 kann ein Nachgärer 11 nachgeordnet werden. Insoweit wird jedoch auf hinlänglich bekannte Einrichtungen von Biogasanlagen verwiesen.

In dem Fermenter 10 wird das Biogas aus biologischem Rohstoff unter Ausschluss von Sauerstoff erzeugt. Dabei kann der Fermenter 10 mit einer kontinuierlichen Vergärung betrieben werden, d. h., dass den Prozessen in regelmäßigen Abständen Substrat zugeführt und Biogas sowie Gärrest entnommen wird.

Der Gärrest wird sodann einem Separator 16 zugeführt, bei dem es sich z. B. um eine Zentrifuge oder eine Schneckenpresse handeln kann. Mittels des Separators 16 werden aus dem Gärrest die Substanzen abgeschieden (Pfeil 15), die nicht vergärbar sind. Hierbei handelt es sich um feste Stoffe, wie Stroh oder Holz, die vor allem Zellulose und Lignozellulose enthalten.

Die flüssige Fraktion (Fugat) wird über eine Leitung 18 einem ersten Behältnis 20 wie Belebungsbecken zugeführt, in dem ein ein- oder mehrstufiger aerober Prozess durchgeführt wird, um einen weiteren Abbau zu erreichen. Über eine Leitung 19 kann die in dem Belebungsbecken 20 homogenisierte Flüssigkeit einer Einrichtung zum Trennen Feststoff Flüssigkeit wie Schrägfilter 35 zugeführt werden, um entwässerten Schlamm, der einen Trockengehalt im Bereich zwischen 8 % und 25 % aufweisen kann, über eine Leitung 22 dem Fermenter 10 zuzuführen.

Die Erfindung zeichnet sich insbesondere dadurch aus, dass dem Belebungsbecken 20, in dem die aerobe Behandlung erfolgt, ein Absetzbecken 21 wie Schrägklärer nachgeschaltet ist. Die in dem Belebungsbecken 20 anfallende Suspension wird dem Absetzbecken 21 zugeführt, so dass sich Feststoffe abscheiden und Wasser übersteht. Dieses überstehende Wasser kann dem Belebungsbecken 20 über eine Leitung 32 zugeführt werden, wobei sich das Verhältnis von der dem Belebungsbecken 20 zugeführten wässrigen Fraktion zu dem zugeführten überstehenden Wasser aus dem Absetzbecken 21 wie 1 : 1 bis 1 : 7, insbesondere in etwa 1 : 5 verhalten sollte, ohne dass die entsprechenden Zahlenwerte einschränkend zu verstehen sind. Durch das Zuführen des Wassers in das Belebungsbecken 20 werden die in der wässrigen Fraktion nicht gelösten Stoffe, also Trockenstoffe, nur wenig oxidiert.

Es besteht jedoch auch die Möglichkeit, dass überstehendes Wasser aus den Absetzbecken 21 über die Leitung 32, 34 unmittelbar zum Beispiel zum Versickern in einem Untergrund, zum Verregnen, zum Einleiten in eine Kläranlage oder in ein Gewässer abgeführt werden.

Um in dem Absetzbecken 21 feinste Partikel abzuscheiden, vorzugsweise Partikel einer Größe zwischen 1 µm und 50 µm, wird dem Absetzbecken 21 ein die Sedimentation beeinflussendes Hilfsmittel zugegeben, und zwar in gegebenenfalls verschiedenen Höhen des Absetzbeckens 21, das z. B. eine zylinderförmige Geometrie aufweisen kann. Das Hilfsmittel wird im Absetzbecken 21 dem überstehendem Fluid 23 zugeführt. Durch die Pfeile 26 soll angedeutet werden, dass das Hilfsmittel in verschiedenen Höhen des überstehenden Wassers 23 in das Absetzbecken 21 eingeleitet werden kann. Ferner besteht die Möglichkeit, in dem Schrägklärer, also dem Absetzbecken 21 das überstehende Wasser 23 im Kreislauf zu führen, um eine bessere Durchmischung mit den Additiven und damit optimale Flockenausbildung zu erreichen. Hierdurch erfolgt eine Einsparung.

Das Sediment 28 kann über eine Leitung 30 dem Fermenter 10 wieder zugeführt werden. Erwähntermaßen kann über die Leitung 32 das überstehende Wasser 23 dem Belebungsbecken 20 zugeführt wird. Alternativ besteht die Möglichkeit, das überstehende Wasser 23 unmittelbar abzuleiten (Pfeil 24).

Das Sediment kann des Weiteren der im Ausführungsbeispiel als Schrägfilter 35 ausgebildeten Trenneinrichtung zugeführt werden, in der eine Feststoff-Flüssigkeitstrennung erfolgt. Der dabei anfallende Schlamm wird bzw. kann über die Leitung 22 dem Fermenter 10 zugeführt werden. Es besteht jedoch auch die Möglichkeit, dass das Sediment unmittelbar über die Leitung 30 dem Fermenter 10 zugeführt wird.

Über die Zuleitung 26 bzw. Zuleitungen 26 wird erwähntermaßen das Hilfsmittel dem in dem Absetzbecken 21 überstehenden Fluid bzw. Wasser 23 zugeführt. Hierbei handelt es sich insbesondere um ein Metallsalz, wie insbesondere Aluminium und/oder Eisenchlorid oder Sulfat. Auch Polymere wie kationische oder anionische Polyacrylamidderivate können verwendet werden. Eine Mischung dieser ist gleichfalls möglich.

Das Flockungshilfsmittel wird von einem Behältnis 27 über die Leitung 29 an die Zuleitungen 26 abgegeben. Dabei kann dem Hilfsmittel über eine Leitung 31 Klarwasser von der im Ausfiihrungsbeispiel als Schrägfilter 25 ausgebildeten Einrichtung zugeführt werden, in der eine Feststoff-Flüssigkeitstrennung erfolgt.

Bevorzugterweise wird als Hilfsmittel eine Mischung verwendet, die Aluminiumhydroxidchlorid enthält, wobei gegebenenfalls kationische und/oder anionische Stärkederivate zugesetzt sein können. Das Aluminiumhydroxidchlorid sollte eine Basizität zwischen 25 % und 95 % aufweisen. Der Gewichtsanteil in der Mischung von Aluminiumhydroxidchlorid sollte mehr als 40 Gew.-% betragen, insbesondere im Bereich zwischen 80 Gew.-% und 97 Gew.-%, liegen. Der Gewichtsanteil der kationischen und/oder anionischen Stärkederivate in der Mischung sollte 0 Gew.-% bis 30 Gew.-%, insbesondere 5 Gew.-% bis 10 Gew.-%, betragen. Die Gesamtsumme der Bestandteile ergeben 100 Gew.-%.

Durch die Hilfsmittel werden erwähntermaßen feinste Partikel im Absetzbecken 21 abgeschieden.

Die erfindungsgemäße Lehre ermöglicht, dass in dem Fugat enthaltende NH₄⁺-Anteil durch speziell konditionierte Mikroorganismen in dem Behältnis bzw. Belebungsbecken 20 zu elementarem Stickstoff umgewandelt werden. Die Mikroorganismen stellen durch ihren Stickstoff-Umsatz des Weiteren sicher, dass CSB, TOC und andere Parameter so stark reduziert werden, dass gereinigtes Wasser über eine Leitung 24 abgeführt werden kann. Das Wasser kann im Untergrund versickert oder verregnet, in eine Kläranlage oder im günstigsten Fall sogar direkt in ein Oberflächengewässer eingeleitet werden.

Das dem Fermenter 10 entnommene Biogas wird über eine Leitung 33 einem Blockheizkraftwerk 36 zugeführt, mittels dessen Strom und Wärme erzeugt werden.

Aufgrund der erfindungsgemäßen Lehre bedarf es dann, wenn im Vergleich zu bekannten Anlagen eine gleiche Menge an Strom und Wärme erzeugt werden soll, weniger Substrat, das über die Leitung 12 dem Fermenter 10 zugeführt wird. Wird die gleiche Menge wie bei bekannten Anlagen zugegeben, so ist eine höhere CH₄-Menge erzielbar, also ein höherer Gewinn an Strom und Wärme.

Unabhängig hiervon wird durch das Rückführen des Schlamms der CH₄-Gehalt im Biogas erhöht.

Wie der Verfahrensablauf gemäß der Fig. verdeutlicht, besteht die Möglichkeit, dass vor der biologischen Stufe in dem Behältnis 20 ein Speicher oder Lager 25 vorgesehen ist, in dem zunächst die wässrige Fraktion 18 gesammelt wird, um sodann dem biologischen Abbau in dem Behältnis 20 unterzogen zu werden. Dabei können über einen oder mehrere Anschlüsse 28 Additive zugegeben werden, um den biologischen Abbau zu beschleunigen.

Ferner ergibt sich aus der Zeichnung, dass von einem Tank 17 Sauerstoff in das Belebungsbecken 20 eingetragen werden kann.

## Patentansprüche

1. Verfahren zum Betreiben einer Biogasanlage umfassend zumindest einen Fermenter (10), in dem Biogas durch Vergärung von dem Fermenter zugeführtem Substrat erzeugt wird, einen dem Fermenter nachgeordneten Gärrest zuzuführenden Separator (16) zum Separieren von insbesondere unvergärtem Feststoff und Gärresten in wässriger Fraktion, wobei die wässrige Fraktion des Gärrestes in einem Belebungsbecken (20) nachbehandelt wird, und in dem Belebungsbecken anfallende Suspension dem nachgeschalteten Absetzbecken (21) zugeführt wird,
**dadurch gekennzeichnet,**
**dass** in dem Absetzbecken (21) vorhandenes überstehendes Wasser (23) vollständig oder teilweise dem Belebungsbecken (20) zugeführt wird.

2. Verfahren nach Anspruch 1,
**dadurch gekennzeichnet,**
**dass** bei der biologischen Nachbehandlung entstandener oder aus der biologischen Nachbehandlung nach erfolgter Filtration gewonnener Schlamm dem Fermenter (10) zugeführt wird.

3. Verfahren nach Anspruch 1 oder 2,
**dadurch gekennzeichnet,**
**dass** bei der Filtration angefallenes Filtrat und/oder bei der Sedimentation im Absetzbecken (21) entstehendes überstehendes Wasser (23) zum Versickern im Untergrund, zum Verregnen, zum Einleiten in eine Kläranlage oder in ein Gewässer verwendet wird.

4. Verfahren nach zumindest einem der vorhergehenden Ansprüche,
**dadurch gekennzeichnet,**
**dass** das Verhältnis von der dem Belebungsbecken zugeführten wässrigen Fraktion zu dem zugeführten überstehenden Wasser (23) aus dem Absetzbecken (21) sich verhält wie 1 : 1 bis 1 : 7, insbesondere in etwa 1 : 5.

5. Verfahren nach zumindest einem der vorhergehenden Ansprüche,
**dadurch gekennzeichnet,**
**dass** dem Fermenter (10) Schlamm, insbesondere filtrierter Schlamm, mit einem Trockengehalt mit 8 % ≤ TS ≤ 25 %, insbesondere 10 % ≤ TS ≤ 20 %, zugeführt wird.

6. Verfahren nach zumindest einem der vorhergehenden Ansprüche,
**dadurch gekennzeichnet,**
**dass** dem Fermenter (10) im Absetzbecken (21) angefallenes Sediment mit einem Trockengehalt zwischen 3 % ≤ TS ≤ 10 %, insbesondere 4 % ≤ TS ≤ 8 %, zugeführt wird.

7. Verfahren nach zumindest einem der vorhergehenden Ansprüche,
**dadurch gekennzeichnet,**
**dass** die wässrige Fraktion vor der biologischen Nachbehandlung gelagert wird.

8. Verfahren nach zumindest einem der vorhergehenden Ansprüche,
**dadurch gekennzeichnet,**
**dass** der wässrigen Fraktion für die biologische Nachbehandlung zumindest ein Additiv zugegeben wird.

9. Verfahren nach zumindest einem der vorhergehenden Ansprüche,
**dadurch gekennzeichnet,**
**dass** dem überstehenden Wasser (23) im Absetzbecken (21) ein Additiv bzw. Flockungshilfsmittel zuzugeben wird.

10. Verfahren nach Anspruch 1 zur Feststoffsedimentation feinster Partikel, vorzugsweise Partikel einer Größe zwischen 1 µm und 50 µm, in dem Absetzbecken (21) mit in diesem vorhandenem Sediment (28) und über diesem überstehendem Fluid (23),
**dadurch gekennzeichnet,**
**dass** in das überstehende Fluid (23) zumindest ein die Sedimentation beeinflussendes Hilfsmittel zugegeben wird.

11. Verfahren nach zumindest Anspruch 10,
**dadurch gekennzeichnet,**
**dass** in verschiedenen Höhen des Behältnisses, insbesondere zylinderförmiger Geometrie, das Hilfsmittel bzw. eine das Hilfsmittel enthaltende Lösung dem überstehenden Fluid (23) zugeführt wird und/oder dass zur Durchmischung des überstehenden Fluids mit dem Hilfsmittel das überstehende Fluid im Kreislauf geführt führt.

12. Verfahren nach zumindest Anspruch 10 oder 11,
**dadurch gekennzeichnet,**
**dass** als Hilfsmittel oder für das Hilfsmittel zumindest eine Substanz aus der Gruppe Metallsalz, insbesondere Aluminium- und/oder Eisensalz wie Chloride oder Sulfate, und/oder Polymere wie kationische und/oder anionische Polyacrylamidderivate verwendet wird.

13. Verfahren nach zumindest einem der Ansprüche 10 bis 12,
**dadurch gekennzeichnet,**
**dass** als Hilfsmittel eine Mischung bestehend aus oder enthaltend Aluminiumhydroxidchlorid und/oder kationische und/oder anionische Stärkederivate verwendet wird.

14. Verfahren nach zumindest Anspruch 13,
**dadurch gekennzeichnet,**
**dass** Aluminiumhydroxidchlorid mit einer Basizität zwischen 25 % und 95 % und einem Gewichtsanteil in der Mischung von mehr als 40 Gew.-%, insbesondere zwischen 80 Gew.-% und 97 Gew.-%, und/oder kationische und/oder anionische Stärkederivate mit einem Gewichtsanteil in der Mischung zwischen 0 Gew.-% und 30 Gew.-%, insbesondere 5 Gew.-% bis 10 Gew.-%, verwendet wird.

15. Verfahren nach zumindest einem der vorhergehenden Ansprüche,
**dadurch gekennzeichnet,**
**dass** für die das Hilfsmittel enthaltende Mischung Klarwasser verwendet wird, das bei der Trennung Feststoff-Flüssigkeit in einer Einrichtung (35) gewonnen wird, dem Suspension aus dem Belebungsbecken (20) und/oder Sediment (28) zugeführt wird.

## Claims

1. A method for operating a biogas plant comprising at least one fermenter (10), in which biogas is generated by the fermentation of substrate fed to the fermenter; and a separator (16) downstream of the fermenter and supplying fermentation residue for separation of in particular unfermented solid matter and fermentation residues in an aqueous fraction, wherein the aqueous fraction of the fermentation residue is post-treated in an aeration tank (20), and the suspension obtained in the aeration tank is fed to the downstream settling tank (21),
**characterized in**
**that** overlaying water (23) present in the settling tank (21) is completely or partially fed to the aeration tank (20).

2. The method according to claim 1,
**characterized in**
**that** sludge resulting from biological post-treatment or obtained from biological post-treatment is fed to the fermenter (10) after completion of filtration.

3. The method according to claim 1 or 2,
**characterized in**
**that** filtrate obtained during filtration and/or overlaying water (23) resulting from sedimentation in the settling tank (21) is used for seepage into the ground, for watering, or for discharge into a treatment plant or into a watercourse.

4. The method according to at least one of the preceding claims,
**characterized in**
**that** the ratio of the aqueous fraction fed into the aeration tank to the overlaying water (23) fed from the settling tank (21) is 1: 1 to 1: 7, in particular around 1: 5.

5. The method according to at least one of the preceding claims,
**characterized in**
**that** sludge, in particular filtrated sludge, is fed to the fermenter (10) with a dry content of 8 % ≤ TS ≤ 25 %, in particular 10 % ≤ TS < 20 %.

6. The method according to at least one of the preceding claims,
**characterized in**
**that** sediment obtained in the settling tank (21) is fed to the fermenter (10) with a dry content between 3 % ≤ TS ≤ 10 %, in particular 4 % ≤ TS ≤ 8 %.

7. The method according to at least one of the preceding claims,
**characterized in**
**that** the aqueous fraction is held in storage prior to biological post-treatment.

8. The method according to at least one of the preceding claims,
**characterized in**
**that** at least one additive is added to the aqueous fraction for biological post-treatment.

9. The method according to at least one of the preceding claims,
**characterized in**
**that** an additive or flocculation aid is added to the overlaying water (23) in the settling tank (21).

10. The method according to claim 1 for solid sedimentation of microfine particles, preferably particles of a size between 1 µm and 50 µm, in the settling tank (21) with sediment (28) present therein and overlaying fluid (23) above it,
**characterized in**
**that** at least one agent influencing the sedimentation is added to the overlaying fluid (23).

11. The method according to at least claim 10,
**characterized in**
**that** at various levels of the container, in particular of cylindrical geometry, the agent or a solution containing said agent is fed to the overlaying fluid (23), and/or that the overlaying fluid is circulated to thoroughly mix said overlaying fluid with the agent.

12. The method according to at least claim 10 or 11,
**characterized in**
**that** at least one substance from the group metal salts, in particular aluminium salt and/or iron salt such as chlorides or sulphates, and/or polymers such as cationic and/or anionic polyacrylamide derivatives, is used as the agent or for the agent.

13. Method according to at least one of claims 10 to 12,
**characterized in**
**that** a mixture consisting of or containing aluminium hydroxide chloride and/or cationic and/or anionic starch derivatives is used as the agent.

14. The method according to at least claim 13,
**characterized in**
**that** aluminium hydroxide chloride is used with a basicity between 25 % and 95 % and a proportion by weight in the mixture of more than 40 % by wt., in particular between 80 and 97 % by wt., and/or cationic and/or anionic starch derivatives with a proportion by weight in the mixture of between 0 and 30 % by wt., in particular 5 to 10 % by wt.

15. The method according to at least one of the preceding claims,
**characterized in**
**that** clear water obtained during solid/liquid separation in a facility (35) is used for the mixture containing the agent and is fed to the suspension from the aeration tank (20) and/or sediment (28).

## Revendications

1. Procédé de fonctionnement d'une installation de production de biogaz, comprenant au moins un fermenteur (10) dans lequel du biogaz est produit par fermentation du substrat distribué au fermenteur, un séparateur (16) en aval du fermenteur, alimenté en digestat afin de séparer les matières solides notamment non fermentées et le digestat dans une fraction aqueuse, sachant que la fraction aqueuse du digestat est post-traitée dans un bassin d'aération (20), et que la suspension formée dans le bassin d'aération est distribuée au bassin de décantation (21) en aval,
**caractérisé en ce**
**que** l'eau (23) surnageant, présente dans le bassin de décantation (21) est distribuée complètement ou partiellement au bassin d'aération (20).

2. Procédé selon la revendication 1,
**caractérisé en ce**
**que** les boues formées lors du post-traitement biologique ou obtenues du post-traitement biologique après filtration sont distribuées au fermenteur (10).

3. Procédé selon la revendication 1 ou 2,
**caractérisé en ce**
**que** le filtrat produit lors de la filtration et/ou l'eau (23) surnageant, formée lors de la sédimentation dans le bassin de décantation (21) sont utilisés pour une infiltration dans le sol, pour une pulvérisation ou pour être introduite dans une station d'épuration ou dans un cours d'eau.

4. Procédé selon au moins l'une des revendications précédentes,
**caractérisé en ce**
**que** le rapport entre la fraction aqueuse, distribuée au bassin d'aération et l'eau (23) surnageant, distribuée, provenant du bassin de décantation (21) est compris entre 1 : 1 et 1 : 7, correspond notamment à environ 1 : 5.

5. Procédé selon au moins l'une des revendications précédentes,
**caractérisé en ce**
**que** des boues, notamment des boues filtrées, avec une teneur sèche telle que 8 % ≤ TS ≤ 25 %, notamment 10 % ≤ TS ≤ 20 % sont distribuées au fermenteur (10).

6. Procédé selon au moins l'une des revendications précédentes,
**caractérisé en ce**
**que** des sédiments, produit dans le bassin de décantation (21), avec une teneur sèche telle que entre 3 % ≤ TS ≤ 10 %, notamment 4 % ≤ TS ≤ 8 % sont distribuées au fermenteur (10).

7. Procédé selon au moins l'une des revendications précédentes,
**caractérisé en ce**
**que** la fraction aqueuse est stockée avant le post-traitement biologique.

8. Procédé selon au moins l'une des revendications précédentes,
**caractérisé en ce**
**qu'**au moins un additif est ajouté à la fraction aqueuse pour le post-traitement biologique.

9. Procédé selon au moins l'une des revendications précédentes,
**caractérisé en ce**
**qu'**un additif ou un adjuvant de floculation est distribué à l'eau (23) surnageant dans le bassin de décantation (21).

10. Procédé selon la revendication 1 pour la sédimentation des matières solides de particules très fines, de préférence de particules d'une taille comprise entre 1 µm et 50 µm dans le bassin de décantation (21) avec des sédiments (28) présents dans ce bassin et du fluide (23) surnageant au-dessus de ces sédiments,
**caractérisé en ce**
**qu'**au moins un adjuvant influençant la sédimentation est ajouté au fluide (23) surnageant.

11. Procédé selon au moins la revendication 10,
**caractérisé en ce**
**que** dans différentes hauteurs du conteneur, notamment avec une géométrie en forme de cylindre, l'adjuvant ou une solution contenant l'adjuvant est distribué(e) au fluide (23) surnageant et/ou que le fluide surnageant est conduit dans le circuit pour mélanger le fluide surnageant à l'adjuvant.

12. Procédé selon au moins la revendication 10 ou 11,
**caractérisé en ce**
**qu'**est utilisé comme adjuvant ou pour l'adjuvant, au moins une substance du groupe du sel métallique, notamment du sel d'aluminium et/ou du sel de fer comme des chlorures ou sulfates, et/ou des polymères comme des dérivés de polyacrylamide cationiques et/ou anioniques.

13. Procédé selon au moins l'une des revendications 10 à 12,
**caractérisé en ce**
**qu'**est utilisé comme adjuvant un mélange constitué de ou contenant du chlorure d'hydroxyde d'aluminium et/ou des dérivés d'amidon cationiques et/ou anioniques.

14. Procédé selon au moins la revendication 13,
**caractérisé en ce**
**qu'**est utilisé du chlorure d'hydroxyde d'aluminium avec une basicité comprise entre 25 % et 95 % et un pourcentage en poids dans le mélange de plus de 40 % en poids, compris notamment entre 80 % et 97 % en poids, et/ou des dérivés d'amidon cationiques et/ou anioniques avec un pourcentage en poids dans le mélange compris entre 0 % et 30 % en poids, notamment 5 % et 10 % en poids.

15. Procédé selon au moins l'une des revendications précédentes,
**caractérisé en ce**
**qu'**est utilisée pour le mélange contenant l'adjuvant de l'eau claire qui est obtenue par la séparation matières solides-liquide dans un équipement (35) auquel est distribué de la suspension provenant du bassin d'aération (20) et/ou des sédiments (28).
